# EUROPEAN PATENT APPLICATION

(11) **EP 4 563 698 A1**
(43) Date of publication of application: **04.06.2025**
(21) Application number: 23846248.5
(22) Date of filing: 13.07.2023
(51) Int. Cl.: C12N 9/02, C07K 17/14, C07K 19/00, C12N 11/14, C12N 15/10, C12P 21/00, G01N 27/327, C12N 15/53, C12N 15/62

(54) **ENZYME ELECTRODE, REDUCTION DEVICE, REDUCTION METHOD, MODIFIED PROTEIN, AND PRODUCTION METHOD FOR MODIFIED PROTEIN**

(30) Priority: 26.07.2022 JP 2022118554
(71) Applicant: Panasonic Intellectual Property Management Co., Ltd., Kadoma-shi, Osaka 571-0057 (JP)
(72) Inventor: HATSUGAI Noriyuki, Kadoma-shi, Osaka 571-0057 (JP); WAYAMA Fumiya, Kadoma-shi, Osaka 571-0057 (JP)
(74) Representative: Grünecker Patent- und Rechtsanwälte PartG mbB
(86) International application number: PCT/JP2023/025815
(87) International publication number: WO 2024/024517

(57) **Abstract**

The present disclosure provides, among other features, an enzyme electrode in which an enzyme is immobilized via more appropriate bonds. An enzyme electrode according to the present disclosure includes an electrode and a modified protein having an amino acid sequence obtained by substituting at least one lysine residue in a fusion protein between a thioredoxin reductase and a thioredoxin with a non-lysine amino acid residue, in which the modified protein is immobilized on a surface of the electrode.

## Description

### Technical Field

The present disclosure relates to an enzyme electrode, a reduction device, a reduction method, a modified protein, and a modified protein production method.

### Background Art

A technique of immobilizing an enzyme on an electrode surface through chemical bonding has been available as a technique for immobilizing an enzyme on an electrode. For example, PTL 1 discloses a method for immobilizing a protein on a metal substrate surface by linking a metal-binding peptide serving as a linker to a protein.

Furthermore, a thioredoxin in a reduced state reduces a disulfide bond of a target protein to induce a structural change, and thereby changes the properties of the protein. For example, PTL 2 discloses an allergen inactivation method involving transforming an oxidized thioredoxin into a reduced form through activation of a thioredoxin reductase by electron donation from an electrode, and continuously reducing disulfide bonds of the allergen.

### Citation List

### Patent Literature

PTL 1: Japanese Unexamined Patent Application Publication No. 2017-37014
PTL 2: International Publication No. 2021/261511

### Summary of Invention

### Technical Problem

Some amino acid side chains originally contained in amino acids constituting an enzyme are capable of forming covalent bonds with other molecules and thus can be used for immobilizing the enzyme on an electrode. However, when amino acids constituting the enzyme are used for the immobilization on the electrode, appropriate bonds are not always formed.

Thus, the present disclosure provides, among other features, an enzyme electrode in which an enzyme is immobilized via more appropriate bonds.

### Solution to Problem

An aspect of the present disclosure provides an enzyme electrode including an electrode and a modified protein having an amino acid sequence obtained by substituting at least one lysine residue in a fusion protein between a thioredoxin reductase and a thioredoxin with a non-lysine amino acid residue, in which the modified protein is immobilized on a surface of the electrode.

Another aspect of the present disclosure provides a reduction device including the enzyme electrode described above and a power supply that applies voltage to the electrode, in which a sample containing a target molecule is brought into contact with the enzyme electrode and the target molecule is reduced while applying voltage to the electrode.

Yet another aspect of the present disclosure provides a reduction method including bringing a sample that can contain a target molecule into contact with the enzyme electrode described above and reducing the target molecule while applying voltage to the electrode.

Still another aspect of the present disclosure provides a modified protein including an amino acid sequence obtained by substituting at least one lysine residue in a fusion protein between a thioredoxin reductase and a thioredoxin with a non-lysine amino acid residue.

Another aspect of the present disclosure provides a modified protein production method including preparing a fusion protein between a thioredoxin reductase and a thioredoxin and substituting at least one lysine residue of the fusion protein with a non-lysine amino acid residue.

### Advantageous Effects of Invention

The present disclosure can provide, among other features, an enzyme electrode in which an enzyme is immobilized via more appropriate bonds.

### Brief Description of Drawings

[Fig. 1] Fig. 1 is a diagram illustrating one example of the structure of an allergen inactivation device according to the embodiment.
[Fig. 2] Fig. 2 is a block diagram illustrating one example of the functional structure of the allergen inactivation device according to the embodiment.
[Fig. 3] Fig. 3 is a diagram illustrating the mechanism of the reduction action of a fusion protein according to the embodiment.
[Fig. 4] Fig. 4 is a flowchart illustrating a fusion protein production method according to the embodiment.
[Fig. 5] Fig. 5 is a conceptual diagram of an expression vector of the fusion protein according to the embodiment.
[Fig. 6] Fig. 6 is a conceptual diagram of the fusion protein according to the embodiment.
[Fig. 7] Fig. 7 is a diagram illustrating an amino acid sequence of the fusion protein according to the embodiment.
[Fig. 8] Fig. 8 is a flowchart illustrating one example of the operation of the allergen inactivation device according to the embodiment.
[Fig. 9] Fig. 9 is a graph showing the results of allergen degradation in Example 1.
[Fig. 10] Fig. 10 is a graph showing the results of allergen degradation in Example 2.
[Fig. 11] Fig. 11 is a graph showing the results of allergen degradation in Example 3.
[Fig. 12] Fig. 12 is a graph showing the results of allergen degradation in Comparative Example.

### Description of Embodiment

### (Knowledge leading to the present disclosure)

In order to reductively cleave the disulfide bond of a target molecule by using electrons supplied from the electrode, a molecular species capable of receiving electrons supplied from the electrode and using these electrons as reducing power to cleave the disulfide bond of the target molecule is necessary. It is known in the related art that an enzyme-protein combination of a thioredoxin reductase (that is, thioredoxin reducing enzyme) and a thioredoxin can be used as such a molecular species. A thioredoxin can undergo changes between two states of an oxidized state and a reduced state. A reduced thioredoxin has a function of cleaving a disulfide bond of a target molecule. A thioredoxin that has cleaved the disulfide bond becomes oxidized in exchange of the disulfide bond cleavage, and turns into an oxidized thioredoxin.

A thioredoxin reductase has a function of reducing the oxidized thioredoxin into a reduced thioredoxin by using electrons. Thus, so long as electrons from the electrode can be supplied to the thioredoxin reductase, the oxidized thioredoxin can be transformed into the reduced thioredoxin. In other words, a cycle of transforming an oxidized thioredoxin, which has been formed as a result of cleaving the disulfide bond of the target molecule, into a reduced thioredoxin and again cleaving a disulfide bond of a target molecule therewith can be repeated.

In addition, it is known that a thioredoxin reductase is not good at directly accepting electrons from the electrode and thus the presence of an electron-transporting substance known as an electron mediator (or simply "mediator") is necessary.

Meanwhile, the thioredoxin's function of cleaving disulfide bonds is utilized in cleaving disulfide bonds of allergens (substances that induce allergic reactions) contained in food. Since cleaving the disulfide bond of an allergen can change the three-dimensional structure of the allergen or can promote such a change, it is possible that the allergic reactions would be suppressed. In other words, a technology of utilizing the disulfide bond cleavage process to produce food with lower risks of allergic reactions and yet still containing allergens has been developed. Representative examples of the allergen include prolamins, ovalbumin, and β-lactoglobulin, but the aforementioned features can be effectively applied to any allergen that has disulfide bonds, in particular, disulfide bonds near the molecular surfaces.

The thioredoxin and thioredoxin reductase are preferably immobilized on the electrode so that the thioredoxin and the thioredoxin reductase do not mix into food produced by the aforementioned process. The same applies to the mediator. In immobilizing such an enzyme or protein (hereinafter collectively referred to as a protein) on an electrode, a technique of immobilizing a protein on an electrode by using lysine residues contained in amino acids constituting the protein to induce formation of covalent bonds by amine coupling between the carboxy groups on the electrode and side-chain amino groups in the lysine residues can be employed. However, more than one lysine residues may be contained in a protein. It is difficult to control which of the lysine residues is to undergo amine coupling.

To address this issue, in the present disclosure, at least one lysine residue at a site unsuitable for amine coupling in amino acids constituting a protein is substituted with a non-lysine amino acid, and the protein that underwent this substitution is used in immobilization on the electrode. As a result, the orientation of the protein immobilized on the electrode can be controlled, and thus, for example, a portion of the protein involved in accepting electrons from the mediator and a portion involved in disulfide bond cleavage by the protein can be appropriately oriented. Thus, an enzyme electrode, etc., in which the enzyme is immobilized via more appropriate bonds from the viewpoint of the reaction efficiency can be realized.

### (General outline of present disclosure)

The general outline of the present disclosure is as follows.

According to a first aspect of the present disclosure, there is provided an enzyme electrode that includes an electrode and a modified protein having an amino acid sequence obtained by substituting at least one lysine residue in a fusion protein between a thioredoxin reductase and a thioredoxin with a non-lysine amino acid residue, in which the modified protein is immobilized on a surface of the electrode.

If, for the sake of argument, the lysine residue substituted in the modified protein remains unsubstituted, the presence of that lysine residue would cause inappropriate enzyme (a fusion protein between a thioredoxin reductase and a thioredoxin) bonding. This adverse effect can be advantageously avoided by using the aforementioned enzyme electrode. Thus, the enzyme can be immobilized via more appropriate bonds with less adverse effects of inappropriate enzyme bonding.

According to a second aspect of the present disclosure, there is provided the enzyme electrode of the first aspect in which the non-lysine amino acid residue is an arginine residue.

According to this aspect, the lysine residue is substituted with an amino acid residue having properties relatively similar to the lysine residue, and thus there is an advantage in that the properties of the fusion protein can be easily also maintained in the modified protein after substitution.

According to a third aspect of the present disclosure, there is provided the enzyme electrode of the first or second aspect in which the at least one lysine residue has a side chain oriented outward on a surface of the fusion protein.

According to this aspect, those lysine residues that are highly likely to affect immobilization of the fusion protein on the surface of the electrode can be substituted.

According to a fourth aspect of the present disclosure, there is provided the enzyme electrode of any one of the first to third aspect, in which the thioredoxin reductase includes a first subunit containing an active center involved in electron transfer and a second subunit different from the first subunit, and the at least one lysine residue is contained in amino acid residues constituting the second subunit.

According to this aspect, since the lysine residue contained in the amino acid residues constituting the second subunit is substituted, substitution of the lysine residue at the active center involved in electron transfer can be easily avoided. Thus, there is an advantage in that the activity involved in electron transfer can be easily maintained in the modified protein after substitution as well.

According to a fifth aspect of the present disclosure, there is provided the enzyme electrode of any one of the first to fourth aspects, in which the fusion protein contains a first lysine residue and a second lysine residue, the first lysine residue is substituted with the non-lysine amino acid residue, and the modified protein is immobilized on the surface of the electrode via the second lysine residue.

If, for the sake of argument, the first lysine residue substituted in the modified protein remains unsubstituted, the presence of that first lysine residue would cause inappropriate enzyme bonding, but this adverse effect can be advantageously avoided according to this aspect. More specifically, when the fusion protein is immobilized on the electrode surface at the site of the first lysine residue, the positional relationship between the fusion protein and the electrode surface becomes inappropriate and the efficiency of various reactions between the electrode surface and the fusion protein would be degraded; however, this issue can be avoided.

According to a sixth aspect of the present disclosure, there is provided the enzyme electrode of the fifth aspect, in which one of the second lysine residue is disposed on a side far from the thioredoxin in the thioredoxin reductase.

According to this aspect, the modified protein can be immobilized on the electrode surface while being on the side far from the thioredoxin in the thioredoxin reductase.

According to a seventh aspect of the present disclosure, there is provided the enzyme electrode of the fifth or sixth aspect, in which one of the second lysine residue is disposed on a side closer to an active center involved in electron transfer in the thioredoxin reductase.

According to this aspect, the modified protein can be immobilized on the electrode surface on the side closer to the active center involved in electron transfer in the thioredoxin reductase.

According to an eighth aspect of the present disclosure, there is provided a reduction device including the enzyme electrode of one of the first to seventh aspects and a power supply that applies voltage to the electrode, in which a sample containing a target molecule is brought into contact with the enzyme electrode and the target molecule is reduced while applying voltage to the electrode.

According to this reduction device, the target molecule can be reduced while achieving the same effects as those of the enzyme electrodes described above.

According to a ninth aspect of the present disclosure, there is provided a reduction method including bringing a sample that can contain a target molecule into contact with the enzyme electrode of any one of the first to seventh aspects and reducing the target molecule while applying voltage to the electrode.

According to this reduction method, the same effects as those of the reduction device can be achieved.

According to a tenth aspect of the present disclosure, there is provided a modified protein including an amino acid sequence obtained by substituting at least one lysine residue in a fusion protein between a thioredoxin reductase and a thioredoxin with a non-lysine amino acid residue.

According to this modified protein, more appropriate bonds can be formed when the protein is immobilized on the electrode surface in the enzyme electrode.

According to an eleventh aspect of the present disclosure, there is provided a modified protein production method including preparing a fusion protein between a thioredoxin reductase and a thioredoxin and substituting at least one lysine residue of the fusion protein with a non-lysine amino acid residue.

According to this modified protein production method, a modified protein that can form more appropriate bonds when the protein is immobilized on the electrode surface in the enzyme electrode can be produced.

It should be noted here that these comprehensive or specific aspects may be realized through any system, method, device, integrated circuit, computer program, or computer-readable recording medium such as CD-ROMs, or may be realized by any desired combination of systems, methods, devices, integrated circuits, computer programs, and recording media.

An embodiment will now be specifically described with reference to the drawings.

The embodiment described below are all general or specific examples. The numerical figures, shapes, materials, constituent elements, arrangement positions and connection modes of constituent elements, steps, orders of steps, etc., disclosed in the embodiment below are merely examples and do not limit of the claims. In addition, of the constituent elements in the embodiment below, those which are not recited in independent claims representing the broadest concepts are described as optional constituent elements. Moreover, the drawings are not necessarily faithful representations. In the drawings, features that are substantially identical are represented by the same reference signs, and the descriptions therefor may be omitted or simplified to avoid redundancy.

In the present disclosure, the terms that indicate relationships between elements, such as parallel and perpendicular, the terms that indicate the shapes of the elements, such as rectangular, and the numerical figures not only have the strict meanings but also include substantially equivalent ranges, for example, differences of about several percent.

In the drawings of the present disclosure, broken lines indicate features that are not visible from the surface or region boundaries.

### (Embodiment)

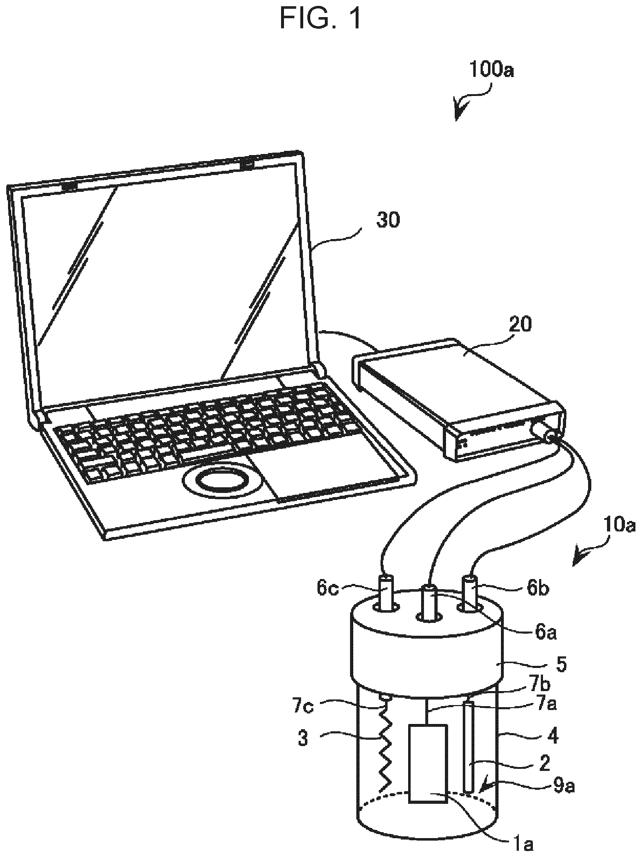

An embodiment will now be specifically described with reference to Figs. 1 to 8.

### [Target molecule reduction device]

### [1. General description]

First, an allergen inactivation device according to the embodiment is generally described with reference to Fig. 1. Fig. 1 is a diagram illustrating one example of the structure of the allergen inactivation device according to the embodiment.

An allergen inactivation device 100a is a device that continuously inactivates allergens by supplying electrons to a fusion protein (modified protein after amino acid substitution described in detail below) inactivated by reducing allergens so that the fusion protein that has lost its reducing power is repeatedly activated. **In** other words, the allergen inactivation device 100a is one example of the reduction device. The allergen inactivation device 100a can adjust the travelling speed and energy amount of electrons supplied to the fusion protein from the electrode (working electrode 1a) by controlling the voltage to be applied to the electrode (working electrode 1a) from a power supply 20.

Note that, as mentioned above, an allergen is a component that induces allergic reactions (or has an allergic activity). An allergen has a site (specific binding site) that specifically binds an antibody (for example, IgE antibody) of a person having allergy. Inactivating an allergen means weakening the allergenicity of the allergen, and, for example, means that the IgE antibody comes to exhibit a lower ability to recognize the specific binding site of the allergen due to changes in the structure of the specific binding site of the allergen (for example, the interaction between the amino acid residues).

For example, the allergen to be targeted in the inactivation according to the embodiment has a disulfide bond. The allergen having a disulfide bond is, for example, an allergenic protein. As described above, a disulfide bond is a very strong bond and is not easy to cleave even with heat, acids, or enzymes (for example, digestive enzymes in the stomach). Accordingly, an allergen having a disulfide bond has a high possibility of inducing allergy. When an allergen has a disulfide bond, reducing the allergen means reducing the disulfide bond in the allergen. The details of reducing the allergen are described below.

### [2. Structure]

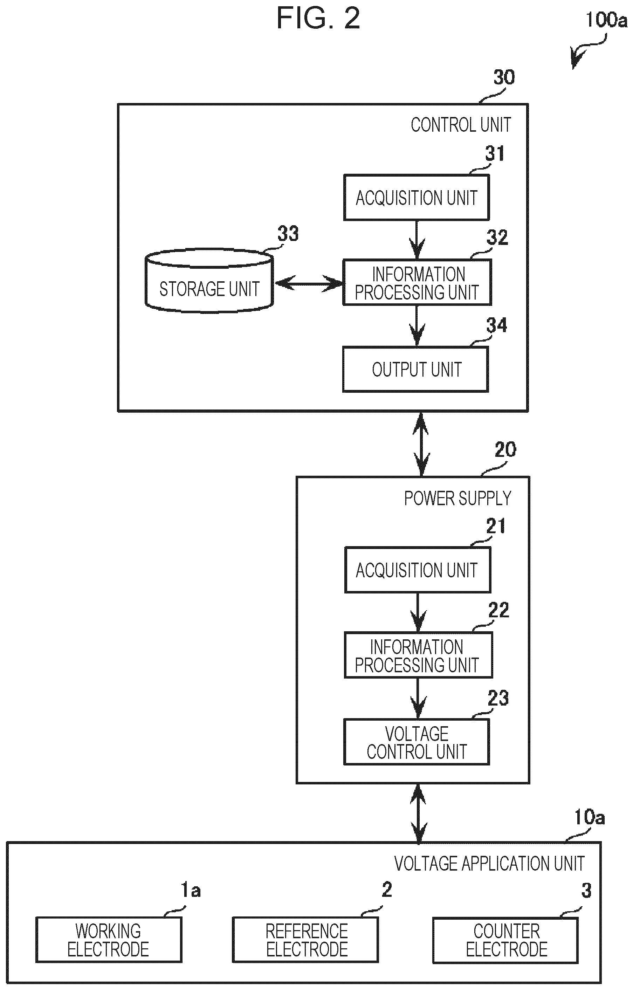

Next, the structure of the allergen inactivation device 100a according to the embodiment is described with reference to Figs. 1 and 2. Fig. 2 is a block diagram illustrating one example of the functional structure of the allergen inactivation device 100a according to the embodiment.

The allergen inactivation device 100a according to the embodiment is equipped with an electrode (working electrode 1a) for supplying electrons to a fusion protein that reductively inactivates the allergen under application of voltage, a power supply 20 that applies voltage to the electrode (working electrode 1a), and a controller 30 that controls the voltage application of the power supply 20. Note that the electrode (hereinafter may be simply referred to as the working electrode 1a) that supplies electrons to the fusion protein constitutes a part of a voltage application unit 10a.

### [Voltage application unit]

The voltage application unit 10a supplies electrons from the electrode (working electrode 1a) to the fusion protein. The voltage application unit 10a is, for example, a three-electrode cell equipped with the working electrode 1a, a reference electrode 2, a counter electrode 3, a cell 4, a lid 5, terminals 6a, 6b, and 6c, and leads 7a, 7b, and 7c. Alternatively, the voltage application unit 10a may be, for example, a two-electrode cell equipped with a working electrode 1a and a counter electrode 3.

The working electrode 1a is an electrode that electrochemically responds sensitively to trace components in a sample solution 9a on the electrode surface. A fusion protein and a mediator (described below) are immobilized on the working electrode 1a. The counter electrode 3 is an electrode that sets the potential difference with respect to the working electrode 1a. The working electrode 1a and the counter electrode 3 are composed of an electrically conductive substance. The electrically conductive substance may be, for example, a carbon material, a conductive polymer material, a semiconductor, or a metal. Examples of the carbon material include carbon nanotubes, Ketjen black (registered trademark), glassy carbon, graphene, fullerene, carbon fibers, carbon fabrics, and carbon aerogel. Examples of the conductive polymer material include polyaniline, polyacetylene, polypyrrole, poly(3,4-ethylenedioxythiophene), poly(p-phenylenevinylene), polythiophene, and poly(p-phenylene sulfide). Examples of the semiconductor include silicone, germanium, indium tin oxide (ITO), titanium oxide, copper oxide, and silver oxide. Examples of the metal include gold, platinum, silver, titanium, aluminum, tungsten, copper, iron, and palladium. Here, for example, the working electrode 1a is a glassy carbon electrode, and the counter electrode 3 is a platinum electrode. Note that the electrically conductive substance may be any electrically conductive substance that remains undecomposed by its own oxidation reaction. The electrically conductive substance is not limited in particular.

The reference electrode 2 is an electrode that does not react with components in the sample solution 9a and maintains a steady potential, and is used so that the potential difference between the working electrode 1a and the reference electrode 2 is controlled to a steady level by the power supply 20. Here, the reference electrode 2 is a silver/silver chloride electrode.

The cell 4 is a retainer that retains the sample solution 9a containing an allergen. The sample solution 9a contains at least an allergen and is in contact with the working electrode 1a.

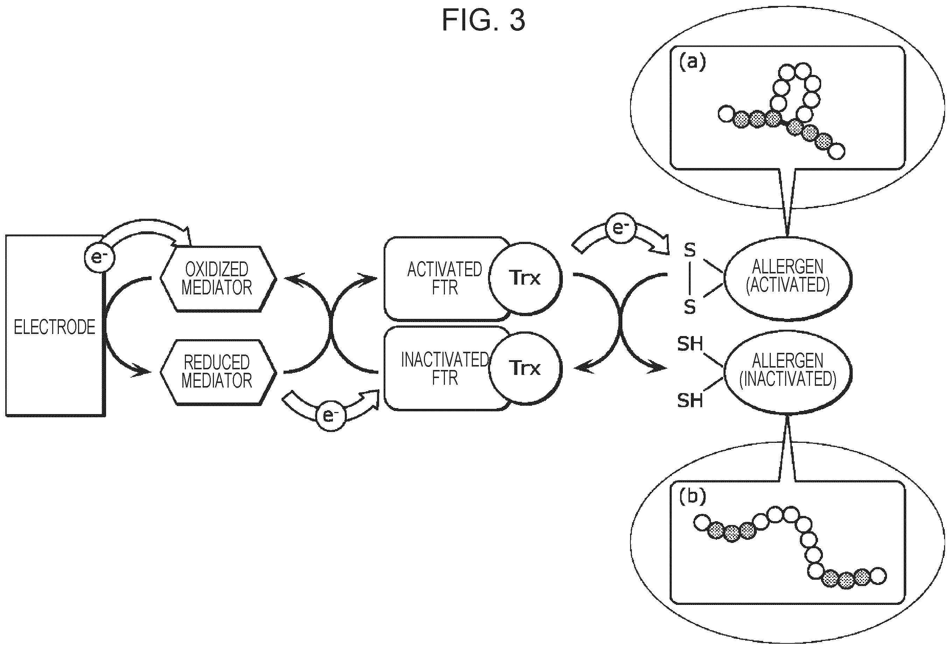

Here, the mechanism of the allergen reduction action of the fusion protein is described with reference to Fig. 3. Fig. 3 is a diagram illustrating the mechanism of the reduction action of the fusion protein according to the embodiment. The electron transfer reaction is a reaction accompanied by transfer of electrons and is a so-called a redox reaction. Each of the components present in the reaction system is reduced by receiving electrons and is oxidized by donating electrons. Thus, each component has two forms of an oxidized form (ox) and a reduced form (red).

For example, as illustrated in Fig. 3, the allergen has a disulfide bond (-S-S-). Examples of the allergenic proteins that have disulfide bonds (hereinafter may be simply referred to as allergenic proteins) include those derived from food such as beans, wheat, milk, seafood, eggs, and rice, environments such as pollen, animals, nematodes, filamentous bacteria, molds, and ticks, and animals such as animal furs and dandruffs.

The disulfide bond in the allergen is reduced to thiol groups (-SH) as electrons are donated from an activated FTR. Fig. 3 schematically illustrates, above the allergen (activated), a portion (a) containing a disulfide bond of the allergen before reduction. Fig. 3 schematically illustrates, below the allergen (inactivated), a portion (b) in the allergen after reduction corresponding to (a) above. Each of open circles illustrated in (a) and (b) indicates one amino acid residue, and each of hatched circles indicates an amino acid residue that the IgE antibody recognizes (epitope). As illustrated in (a) and (b), when the disulfide bond of the allergen is reduced, the looped portion of the amino acid sequence becomes unlooped, and thus a different amino acid sequence is inserted in the middle of the amino acid sequence recognized by the IgE antibody. As a result, there is a higher possibility that the IgE antibody could no longer recognize the specific binding site. Thus, the allergenicity of the allergen is weakened.

Note here that although a looped portion is described as an example in (a) and (b) above, the portion is not limited thereto. In a three-dimensional structure of a protein, a functional portion is formed as amino acid residues remote from each other in an amino acid sequence of a peptide chain of the protein come close to each other. Thus, when the disulfide bond is reduced, the link between secondary structures of the protein that has been formed by the disulfide bond breaks, and the functional portion formed by the linking of the secondary structures is no longer maintained. To be more specific, a disulfide bond is a bond that links secondary structures of the proteins to each other and strengthens the three-dimensional structure of the protein. Thus, when the disulfide bond is cleaved, the link between the secondary structures breaks, and the flexibility (fluctuation) of the three-dimensional structure of the allergenic protein increases. As a result, it becomes difficult to maintain the functional portion of the allergenic protein (for example, a conformational epitope), and thus the possibility of the IgE antibody being unable to recognize the specific binding site of the allergenic protein increases.

In addition, since the reduction of the disulfide bond increases the flexibility (fluctuation) of the three-dimensional structure of the allergenic protein, digestive enzymes smoothly act on the cleaved sites of the peptide chains of the allergenic protein cleaved by the digestive enzymes, and thus the allergenic protein can be more smoothly digested by the digestive enzymes.

In the present embodiment, a fusion protein between a thioredoxin reductase and a thioredoxin (Trx) fused with a linker and before amino acid substitution is used as a base, some of the lysine residues in this fusion protein are substituted with non-lysine amino acid residues, and the resulting substituted fusion protein is used as the fusion protein. In the description below, the properties that remain substantially unchanged before and after the substitution of the lysine residues are described and thus the terms thioredoxin reductase and thioredoxin before substitution are used for the sake of convenience.

A thioredoxin is a low-molecular-weight redox protein that has an amino acid sequence active site motif Trp (tryptophan)-Cys (cysteine)-Gly (glycine)-Pro (proline)-Cys (cysteine). A thioredoxin takes two forms (i.e., a reduced form and an oxidized form) depending on the redox state of thiol groups in 2 Cys in the active site.

A thioredoxin reductase is an enzyme that catalyzes reactions of reducing a thioredoxin. A thioredoxin reductase donates electrons to a thioredoxin. More specifically, a thioredoxin reductase donates electrons supplied from the working electrode 1a to an oxidized thioredoxin. The thioredoxin reductase may have electrons donated from the working electrode 1a via a mediator. The thioredoxin reductase is, for example, an enzyme that catalyzes reduction of an oxidized thioredoxin, including (i) a nicotinamide adenine dinucleotide phosphate (NADPH)-thioredoxin reductase or a ferredoxin-thioredoxin reductase (FTR in the drawing).

A linker is a linker peptide for fusing the aforementioned thioredoxin reductase and thioredoxin to prepare a fusion protein before amino acid substitution. The linker peptide fuses a thioredoxin reductase with a thioredoxin so that the redox-active disulfide of the thioredoxin can interact with the redox-active disulfide of the thioredoxin reductase.

As illustrated in Fig. 3, when a fusion protein is used for the inactivation of an allergen, the fusion protein donates electrons to the allergen upon accepting electrons donated from the electrode to the mediator. Then the disulfide bond of the allergen is reduced to thiol groups as electrons are donated from the activated fusion protein. As a result, the allergen is inactivated.

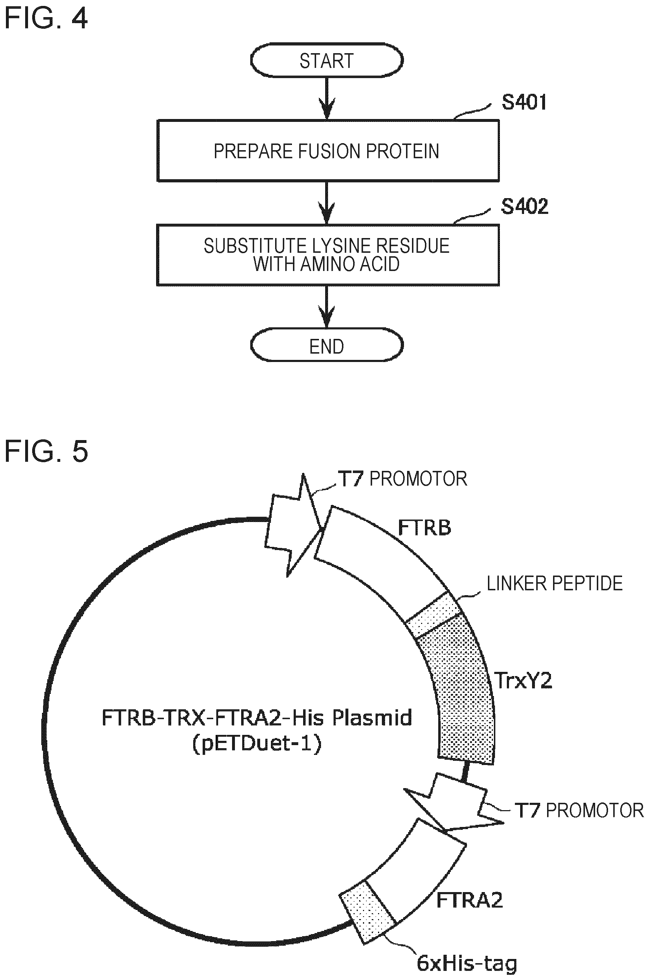

The details of the fusion protein described above and its production method will now be described with reference to Figs. 4 to 8. Fig. 4 is a flowchart illustrating a fusion protein production method according to the embodiment. As illustrated in Fig. 4, in producing a fusion protein, a fusion protein before amino acid substitution in which a thioredoxin reductase and a thioredoxin reductase are fused is prepared (S401). Here, preparing a fusion protein means preparing an expression vector that expresses a fusion protein before amino acid substitution.

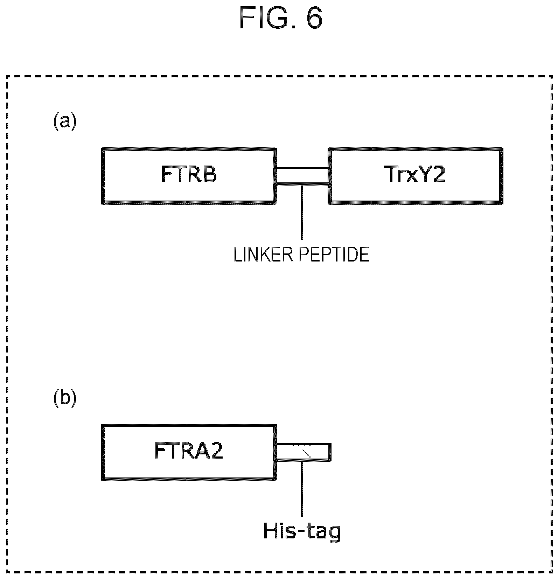

Fig. 5 is a conceptual diagram of the expression vector of the fusion protein according to the embodiment. Fig. 6 is a conceptual diagram of the fusion protein according to the embodiment. Note that, in Fig. 6, a conceptual diagram of a first subunit is illustrated in (a), and a conceptual diagram of a second subunit is illustrated in (b). As illustrated in Fig. 5, a gene (SEQ ID NO: 1) encoding the 32nd to 146th amino acids of FTRB and a gene (SEQ ID NO: 2) encoding 59th to 167th amino acids of TrxY2 were respectively inserted at the 5' side and at the 3' side of a multiple cloning site 1 of an enzyme expression vector (pETDuet-1, available from Novagen). Between these two genes, genes (SEQ ID NOs: 3 to 5) encoding a linker peptide were introduced in necessary units so as to induce expression in an appropriate order. A gene (SEQ ID NO: 6) encoding 73rd to 184th amino acids of FTRA2 and a gene (SEQ ID NO: 7) encoding a histidine tag (His-Tag) were introduced into a multiple cloning site 2. As apparent from Fig. 6 and the description above, the thioredoxin reductase according to the present embodiment is constituted by FTRB which is a first subunit that includes an active center involved in electron transfer, and FTRA2 which is a second subunit that is not integrated (not covalently bonded) with FTRB and has a function of supporting the three-dimensional structure of FTRB.

### Base sequences of inserted genes for preparing expression vector

(1) Gene encoding 32nd to 146th amino acids of FTRB (SEQ ID NO: 1) 
A T G G C G A A A A C G G A A C C G T C G G A G A A A T C A G T A G
A G A T T A T G A G G A A A T T C T C C G A G C A A T A T G C T C G
T C G C T C T G G G A C T T A C T T C T G T G T T G A T A A A G G A
G T T A C T T C A G T C G T T A T T A A G G G T T T G G C T G A G C
A T A A A G A T T C A T A T G G T G C A C C G C T T T G C C C T T G
C A G A C A C T A T G A T G A T A A A G C T G C T G A G G T T G G A
C A A G G C T T T T G G A A T T G T C C G T G T G T T C C A A T G A
G A G A G A G G A A G G A G T G C C A T T G T A T G C T T T T C T T
A A C T C C T G A T A A T G A T T T C G C T G G A A A A G A T C A G
A C G A T T A C A T C G G A T G A A A T A A A A G A A A C T A C A G
C T A A C A T G
(2) Gene encoding 59th to 167th amino acids of TrxY2 (the underlined part indicates a stop codon) (SEQ ID NO: 2)
   [Chem. 1] 
ATGGCAGCAAAGAAGCAAACTTTCAACTCTT
TTGATGATTTGTTGCAGAACTCTGATAAACC
TGTACTTGTAGACTTCTATGCTACTTGGTGT
GGTCCATGTCAGCTTATGGTACCTATACTCA
ATGAAGTGAGTGAAACACTGAAAGATATAAT
TGCAGTTGTGAAAATTGATACTGAGAAGTAC
CCAAGTCTTGCTAACAAATACCAGATTGAGG
CTTTGCCCACATTCATCTTATTCAAAGACGG
CAAACTCTGGGACCGTTTCGAGGGTGCCTTG
CCAGCGAACCAACTCGTTGAGCGTATTGAGA
ATTCTTTGCAAGTGAAACAGTAG
(3) Genes encoding a linker peptide (SEQ ID NOs: 3 to 5)
   (GGGGS)₁ : ggcggcggcggcagc (SEQ ID NO: 3) 
(GGGGS)2: g g c g g c g g c g g c a g c g g c g g c g g c g g
c a g c
(SEQ ID NO: 4)
 
(GGGGS)3: g g c g g c g g c g g c a g c g g c g g c g g c g g
c a g c g g c g g c g g c g g c a g c
(SEQ ID NO: 5)
(4) Gene encoding 73rd to 184th amino acids of FTRA2 (SEQ ID NO: 6) 
ATGGATATTGCTGTGAAATCGGCGGCTTCTGTAG
ACGCGGACGCTGATCTATCGTCATCTACGTCGTT
GGAGACGGAGGAAGACGAGAAAGCGAAGGAGAAG
ATTGGAGCTAGGGTTAGGGTTACGGTTCCGTTGA
AAGTTTACCATGTGGTTCGTGTACCGGAGGTTGA
ATTGATGGGAATGGAAGGTTTTATCAAAGATTAC
GTCGTTCTTTGGAAAGGAAAGAAAATCTCAGCTA
ATCTGCCGTTTAAGGTACAGTTCGTGAAAGAGAT
CGAAGGTCGTGGTCCTGTCAAATTCTTCACGCAT
CTTAAGGAAGACGAGTTCGAGTTAATTGATCCG
(5) Gene encoding a histidine tag (His-Tag) (the underlined part indicates a stop codon) (SEQ ID NO: 7)
   [Chem. 2]
   C A C C A C C A C C A C C A C C A CT G A

Referring back to Fig. 4, next, at least one lysine residue of an amino acid constituting the prepared fusion protein before amino acid substitution is substituted with another amino acid (S402). Here, the amino acid substitution means substituting bases on a gene encoding the fusion protein before amino acid substitution, and when the gene after this substitution is expressed, a substituted fusion protein is generated.

To do this, cloning is performed on an expression vector of the fusion protein before amino acid substitution by using a primer that substitutes lysine residues with non-lysine amino acid residues so as to prepare an expression vector of the intended fusion protein. Here, the primer is designed to substitute the lysine residues with arginine residues that have relatively small impact on the three-dimensional structure after the amino acid substitution.

In selecting lysine residues to be substituted, those lysine residues that have side chains oriented outward on the surface of the fusion protein before amino acid substitution may be selected. Moreover, for example, it is preferable to leave intact (not to substitute) lysine residues that are far from the thioredoxin than from the center of gravity in the thioredoxin reductase so that the thioredoxin is oriented on the side opposite from the electrode. Furthermore, for example, it is preferable to leave intact (not to substitute) lysine residues that are close to the active center involved in electron transfer than from the center of gravity in the thioredoxin reductase so that the active center involved in electron transfer is oriented on the same side as the electrode. Meanwhile, in selecting lysine residue to be substituted, those lysine residues that are involved in the disulfide bond cleaving action, those lysine residues that can notably change the three-dimensional structure of the protein, and those lysine residues that are near (for example, within ten and several angstroms from) the active center involved in donating and accepting electrons should not be selected.

There are four types of designed amino acid substitution, and primers used in respective substitutions each have a primer set of forward (Fwd) and reverse (Rev) complementary strands. Specifically, amino acid substitution is performed by using a K30R/K32R/K34R primer set (SEQ ID NOs: 8 and 9) that substitutes Lys (K) at 30, 32, and 34 positions of FTRA2 with Arg (R), a K104R primer set (SEQ ID NOs: 10 and 11) that substitutes Lys (K) at 104 position of FTRA2 with Arg (R), a K199R primer set (SEQ ID NOs: 12 and 13) that substitutes Lys (K) at 199 position of FTRB-TrxY2 with Arg (R), and a K212R/K215R primer set (SEQ ID NOs: 14 and 15) that substitutes Lys (K) at 212 and 215 positions of FTRB-TrxY2 with Arg (R).

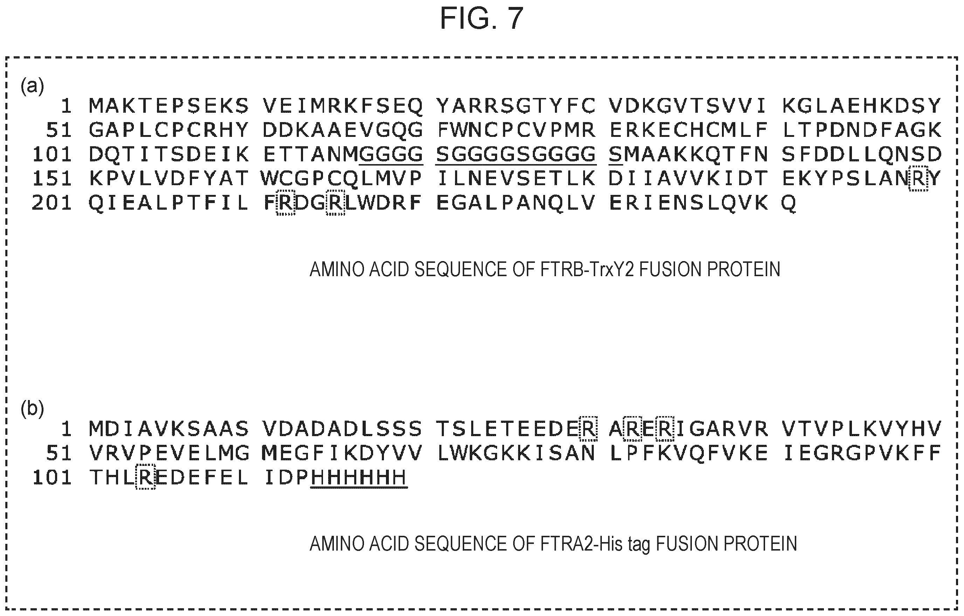

In the present embodiment, following fusion proteins were prepared and used: a fusion protein containing K199R FTRB-TrxY2 (SEQ ID NO: 16), a fusion protein containing K199R/K212R/K215R FTRB-TrxY2 (SEQ ID NO: 17), and a fusion protein containing K30R/K32R/K34R/K104R FTRA2 (SEQ ID NO: 18) and K199R/K212R/K215R FTRB TrxY2. For example, Fig. 7 is a diagram indicating amino acid sequences of a fusion protein containing K30R/K32R/K34R/K104R FTRA2 and K199R/K212R/K215R FTRB-TrxY2. In Fig. 7, the amino acid sequence of the first subunit is indicated in (a), and the amino acid sequence of the second subunit is indicated in (b).

### Base sequences of primers for preparing expression vector

(1) Fwd primer for K30R/K32R/K34R amino acid substitution of FTRA2 (SEQ ID NO: 8) 
GGAGACGGAGGAAGACGAGGCAGCGGCGGAGGCG
ATTGGAGCTAGGGTTAGG
(2) Rev primer for K30R/K32R/K34R amino acid substitution of FTRA2 (SEQ ID NO: 9) 
CCTAACCCTAGCTCCAATCGCCTCCGCCGCTGCC
TCGTCTTCCTCCGTCTCC
(3) Fwd primer for K104R amino acid substitution of FTRA2 (SEQ ID NO: 10) 
TCCTGTCAAATTCTTCACGCATCTTCGTGAAGAC
GAGTTCGAGTTAATTGATC
(4) Rev primer for K104R amino acid substitution of FTRA2 (SEQ ID NO: 11) 
GATCAATTAACTCGAACTCGTCTTCACGAAGATG
CGTGAAGAATTTGACAGGA
(5) Fwd primer for K199R amino acid substitution of FTRB-TrxY2 (SEQ ID NO: 12) 
A A G T A C C C A A G T C T T G C T A A C C G T T A C C A G A T T G
A G G C T T T G C C C
(6) Rev primer for K199R amino acid substitution of FTRB-TrxY2 (SEQ ID NO: 13) 
GGGCAAAGCCTCAATCTGGTAACGGTTAGCAAGA
CTTGGGTACTT
(7) Fwd primer for K212R/K215R amino acid substitution of FTRB-TrxY2 (SEQ ID NO: 14) 
T T G A G G C T T T G C C C A C A T T C A T C T T A T T C C G T G A
C G G C C G T C T C T G G G A C C G T T T C G A G
(8) Rev primer for K212R/K215R amino acid substitution of FTRB-TrxY2 (SEQ ID NO: 15)

C T C G A A A C G G T C C C A G A G A C G G C C G T C A C G G A A T
A A G A T G A A T G T G G G C A A A G C C T C A A

### Amino acid sequences of fusion proteins

(1) K199R FTRB-TrxY2 (the underlined part indicates the arginine residue after substitution) (SEQ ID NO: 16) [Chem. 3] 
Met Ala Lys Thr Glu Pro Ser Glu Lys Ser Val Glu Ile Met Arg Lys
Phe Ser Glu Gln Tyr Ala Arg Arg Ser Gly Thr Tyr Phe Cys Val Asp
Lys Gly Val Thr Ser Val Val Ile Lys Gly Leu Ala Glu His Lys Asp
Ser Tyr Gly Ala Pro Leu Cys Pro Cys Arg His Tyr Asp Asp Lys Ala
Ala Glu Val Gly Gln Gly Phe Trp Asn Cys Pro Cys Val Pro Met Arg
Glu Arg Lys Glu Cys His Cys Met Leu Phe Leu Thr Pro Asp Asn Asp
Phe Ala Gly Lys Asp Gln Thr Ile Thr Ser Asp Glu Ile Lys Glu Thr
Thr Ala Asn Met Gly Gly Gly Gly Ser Gly Gly Gly Gly Ser Gly Gly
Gly Gly Ser Met Ala Ala Lys Lys Gln Thr Phe Asn Ser Phe Asp Asp
Leu Leu Gln Asn Ser Asp Lys Pro Val Leu Val Asp Phe Tyr Ala Thr
Trp Cys Gly Pro Cys Gln Leu Met Val Pro Ile Leu Asn Glu Val Ser
Glu Thr Leu Lys Asp Ile Ile Ala Val Val Lys Ile Asp Thr Glu Lys
Tyr Pro Ser Leu Ala Asn Arg Tyr Gln Ile Glu Ala Leu Pro Thr Phe
Ile Leu Phe Lys Asp Gly Lys Leu Trp Asp Arg Phe Glu Gly Ala Leu
Pro Ala Asn Gln Leu Val Glu Arg Ile Glu Asn Ser Leu Gln Val Lys
Gln
(2) K199R/K212R/K215R FTRB-TrxY2 (the underlined parts indicate the arginine residues after substitution) (SEQ ID NO:17) [Chem. 4] 
Met Ala Lys Thr Glu Pro Ser Glu Lys Ser Val Glu Ile Met Arg Lys
Phe Ser Glu Gln Tyr Ala Arg Arg Ser Gly Thr Tyr Phe Cys Val Asp
Lys Gly Val Thr Ser Val Val Ile Lys Gly Leu Ala Glu His Lys Asp
Ser Tyr Gly Ala Pro Leu Cys Pro Cys Arg His Tyr Asp Asp Lys Ala
Ala Glu Val Gly Gln Gly Phe Trp Asn Cys Pro Cys Val Pro Met Arg
Glu Arg Lys Glu Cys His Cys Met Leu Phe Leu Thr Pro Asp Asn Asp
Phe Ala Gly Lys Asp Gln Thr Ile Thr Ser Asp Glu Ile Lys Glu Thr
Thr Ala Asn Met Gly Gly Gly Gly Ser Gly Gly Gly Gly Ser Gly Gly
Gly Gly Ser Met Ala Ala Lys Lys Gln Thr Phe Asn Ser Phe Asp Asp
Leu Leu Gln Asn Ser Asp Lys Pro Val Leu Val Asp Phe Tyr Ala Thr
Trp Cys Gly Pro Cys Gln Leu Met Val Pro Ile Leu Asn Glu Val Ser
Glu Thr Leu Lys Asp Ile Ile Ala Val Val Lys Ile Asp Thr Glu Lys
Tyr Pro Ser Leu Ala Asn Arg Tyr Gln Ile Glu Ala Leu Pro Thr Phe
Ile Leu Phe Arg Asp Gly Arg Leu Trp Asp Arg Phe Glu Gly Ala Leu
Pro Ala Asn Gln Leu Val Glu Arg Ile Glu Asn Ser Leu Gln Val Lys
Gln
(3) K30R/K32R/K34R/K104R FTRA2 (the underlined parts indicate the arginine residues after substitution) (SEQ ID NO:18)

[Chem. 5]
Met Asp Ile Ala Val Lys Ser Ala Ala Ser Val Asp Ala Asp Ala Asp
Leu Ser Ser Ser Thr Ser Leu Glu Thr Glu Glu Asp Glu Arg
Ala Arg Glu Arg Ile Gly Ala Arg Val Arg Val Thr Val Pro Leu Lys
Val Tyr His Val Val Arg Val Pro Glu Val Glu Leu Met Gly Met Glu
Gly Phe Ile Lys Asp Tyr Val Val Leu Trp Lys Gly Lys Lys Ile Ser
Ala Asn Leu Pro Phe Lys Val Gln Phe Val Lys Glu Ile Glu Gly Arg
Gly Pro Val Lys Phe Phe Thr His Leu Arg Glu Asp Glu Phe Glu Leu
Ile Asp Pro His His His His His His

Referring back to Fig. 3, a mediator is a redox substance that mediates electron migration between the electrode and the fusion protein. The mediator donates electrons to the fusion protein. More specifically, the mediator donates electrons supplied from the working electrode 1a to the fusion protein. Note that the mediator may indirectly supply electrons to an oxidized fusion protein via another redox molecule. The mediator may be any substance that enables electron transfer between the electrode and the fusion protein. The mediator may be selected according to the redox potential of the target molecule (in other words, FTR of the fusion protein here) to which the mediator donates electrons. The mediator may be, for example, a compound having a bipyridine skeleton, a compound having a quinone skeleton, or a compound having a phenylenediamine skeleton. These compounds may be used alone or in combination.

The compound having a bipyridine skeleton may be, for example, a compound having a 2,2'-bipyridine skeleton, a compound having a 2,4'-bipyridine skeleton, a compound having a 4,4'-bipyridine skeleton, or a derivative thereof (for example, a 4,4'-bipyridinium derivative). The compound having a bipyridine skeleton may be a bipyridine compound having a substituent in the bipyridine skeleton (that is, a bipyridine derivative) or a bipyridine compound having no substituents. Examples of the substituent include hydrogen, halogen, a hydroxyl group, a nitro group, a carboxyl group, a carbonyl group, an amino group, an amide group, or a sulfonic acid group, or an alkyl group, aryl group, heteroaromatic alkyl group, or phenyl group substituted therewith. Furthermore, an aromatic ring may be formed between two adjacent substituents. These matters related to the substituent also hold true for the compound having a quinone skeleton and the compound having a phenylenediamine skeleton described below. When the electron mediator is a compound having a bipyridine skeleton, the electron mediator may be, for example, 1,1'-dimethyl-4,4'-bipyridinium (methylviologen), 1-methyl-1'-carboxylmethyl-4,4'-bipyridinium, 1,1'-dicarboxymethyl-4,4'-bipyridinium, 1-methyl-1'-aminoethyl-4,4'-bipyridinium, 1,1'-diaminoethyl-4,4'-bipyridinium, 1-methyl-1'-ethyl-4,4'-bipyridinium, 1-methyl-1'-propyl-4,4'-bipyridinium, 1-methyl-1'-butyl-4,4'-bipyridinium, 1-methyl-1'-pentylhexyl-4,4'-bipyridinium, 1-methyl-1'-hexyl-4,4'-bipyridinium, 1-methyl-1'-heptyl-4,4'-bipyridinium, 1-methyl-1'-octyl-4,4'-bipyridinium, 1-methyl-1'-nonyl-4,4'-bipyridinium, or 1-methyl-1'-decyl-4,4'-bipyridinium, or any of these compounds in which the 1-position methyl group is substituted with an ethyl group.

The compound having a quinone skeleton may be, for example, a compound having a benzoquinone skeleton, a compound having a naphthoquinone skeleton, a compound having an anthraquinone skeleton, or a derivative thereof. The compound having a quinone skeleton may have substituents or no substituents. Since the matters related to the substituent are described above, the description is omitted here. When the electron mediator is a compound having a quinone skeleton, the electron mediator may be, for example, methylbenzoquinone, dimethylbenzoquinone (for example, 2,5-dimethyl-1,4-benzoquinone, 2,3-dimethyl-1,4-benzoquinone, or 2,6-dimethyl-1,4-benzoquinone), 2,3-dichloro-5,6-dicyano-1,4-benzoquinone, 2,3,5,6-tetramethyl-1,4-benzoquinone, 2,3,5,6-tetrachloro-1,4-benzoquinone (chloranil), ubiquinone (CoQ), pyrroloquinoline quinone (PQQ), 1,2-naphthoquinone-4-sulfonic acid, 2-methyl-1,4-naphthoquinone (vitamin K3), 2-hydroxy-1,4-naphthoquinone, 1,2-dihydroxyanthraquinone (alizarin), 1,2,4-trihydroxyanthraquinone (purpurin), or 9,10-phenanthrenequinone. Furthermore, the electron mediator may be, for example, a benzenediol in which ketone groups in the benzoquinone skeleton are substituted with hydroxyl groups, more specifically, hydroquinone in which the ketone groups in 1,4-benzoquinone are substituted with hydroxyl groups (1,4-benzenediol) or resorcinol in which the ketone groups in 1,3-benzoquinone are substituted with hydroxyl groups (1,3-benzenediol).

The compound having a phenylenediamine skeleton may have substituents or no substituents. When the electron mediator is a compound having a phenylenediamine skeleton, the electron mediator may be, for example, p-phenylenediamine, 2,3,5,6-tetramethyl-1,4-phenylenediamine, N,N-dimethyl-p-phenylenediamine, N,N'-diphenyl-p-phenylenediamine (DPPD), N-isopropyl-N'-phenyl-p-phenylenediamine (IPPD), or N-(1,3-dimethylbutyl)-N'-phenyl-p-phenylenediamine (6PPD).

Referring back to Fig. 1, terminals 6a, 6b, and 6c that respectively electrically connect the working electrode 1a, the reference electrode 2, and the counter electrode 3 to the power supply 20 are provided to the lid 5. A lead extends from each terminal to connect the terminal to the battery. The working electrode 1a is connected to the terminal 6a via a lead 7a, the reference electrode 2 is connected to the terminal 6b via a lead 7b, and the counter electrode 3 is connected to the terminal 6c via a lead 7c.

### [Power supply]

The power supply 20 applies voltage to the electrode (working electrode 1a). More specifically, pursuant to the control signal output from the controller 30, the power supply 20 applies voltage between the working electrode 1a and the counter electrode 3 of the voltage application unit 10a and controls the potential difference between the working electrode 1a and the reference electrode 2 to a particular value. For example, the power supply 20 may apply the voltage such that the voltage applied to the working electrode 1a with respect to the reference electrode 2 (0 V) is greater than or equal to -1.0 V and less than or equal to 0 V. Here, the reference electrode 2 is, for example, a silver/silver chloride electrode.

As illustrated in Fig. 2, the power supply 20 is equipped with an acquisition unit 21, an information processing unit 22, and a voltage control unit 23.

The acquisition unit 21 acquires a control signal output from the controller 30. In addition, the acquisition unit 21 may acquire measurement data such as the potential of each of the electrodes in the voltage application unit 10a and the value of the current flowing in the sample solution 9a.

The information processing unit 22 processes information acquired by the acquisition unit 21. For example, upon acquiring the control signal from the acquisition unit 21, the information processing unit 22 outputs the acquired control signal to the voltage control unit 23. When the voltage control unit 23 starts applying voltage to each of the electrodes of the voltage application unit 10a, the information processing unit 22 acquires measurement data such as the potential of each electrode in the voltage application unit 10a and the value of the current flowing in the sample solution 9a acquired from the acquisition unit 21, and derives the voltage to be applied to the working electrode 1a on the basis of the acquired data so that the potential difference between the working electrode 1a and the reference electrode 2 is maintained at a particular value. Then a control signal that controls the voltage applied to the working electrode 1a according to the derived voltage is output to the voltage control unit 23.

The voltage control unit 23 applies voltage to each of the electrodes of the voltage application unit 10a on the basis of the control signal output from the information processing unit 22.

Note that, in Fig. 1, the power supply 20 and the controller 30 are separate; alternatively, the power supply 20 may include the controller 30.

### [Controller]

The controller 30 executes information processing for controlling the voltage application by the power supply 20. The controller 30 is realized by, for example, a processor, a microcomputer, or a dedicated circuit. In Fig. 1, an example in which the controller 30 is a computer device is illustrated.

The controller 30 includes, for example, an acquisition unit 31, an information processing unit 32, a storage unit 33, and an output unit 34.

For example, the acquisition unit 31 acquires information regarding instructions input by the user (hereinafter, referred to as instruction information) and outputs the acquired instruction information to the information processing unit 32.

The information processing unit 32 derives conditions for applying voltage to each of the electrodes of the voltage application unit 10a (hereinafter may also be referred to as voltage application conditions) on the basis of, for example, the instruction information acquired by the acquisition unit 31. The instruction information may be, for example, the type of the allergen, the amount of the sample solution 9a, the process completion time, the clock time of the completion, or the degree of inactivation (reduction) (for example, a percentage or a five-point scale).

Furthermore, the information processing unit 32 may output, to the output unit 34, a control signal that controls the voltage application under the conditions derived from the instruction information, or may output, to the output unit 34, a control signal that controls the voltage application under the voltage application conditions preset by the user.

The output unit 34 outputs the control signal acquired from the information processing unit 32 to the power supply 20.

The storage unit 33 stores data such as instruction information acquired by the acquisition unit 31 and computer programs to be run by the controller 30 (for example, application programs for controlling the power supply 20).

### [3. Operation]

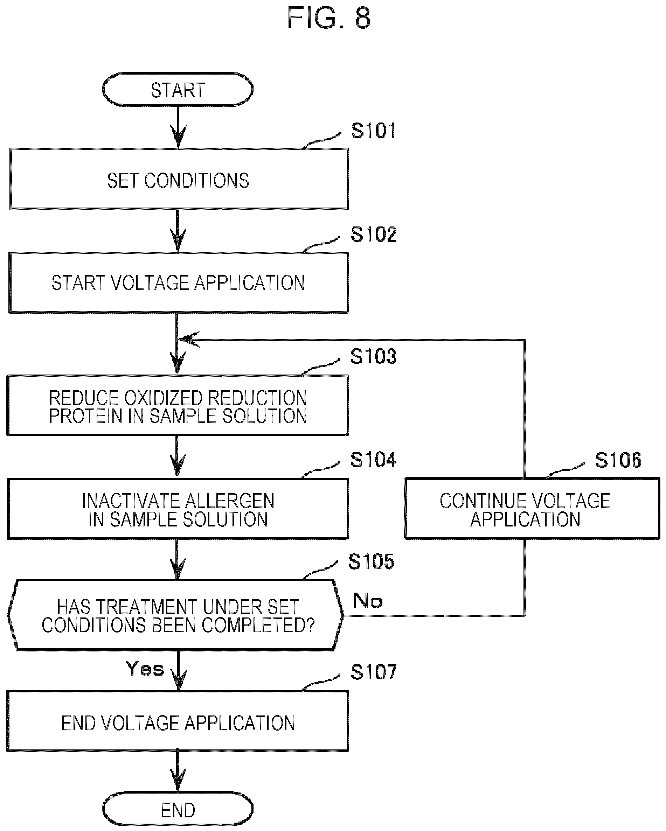

Next, the operation of the allergen inactivation device 100a according to the embodiment is specifically described with reference to Fig. 8. Fig. 8 is a flowchart illustrating one example of the operation of the allergen inactivation device 100a according to the embodiment.

First, a preparation step (not illustrated) before operating the allergen inactivation device 100a is described. For example, the preparation step may be performed by the user. In the preparation step, a sample solution 9a is first prepared. The user introduces a sample that can contain an allergen into the cell 4 of the voltage application unit 10a.

Next, the user inserts electrodes into the sample solution 9a and sets the electrodes to come into contact with the sample solution 9a. The electrodes are a working electrode 1a, a reference electrode 2, and a counter electrode 3. The working electrode 1a is connected to a lead 7a extending from a terminal 6a provided to the lid 5, the reference electrode 2 is connected to a lead 7b extending from a terminal 6b provided to the lid 5, and the counter electrode 3 is connected to a lead 7c extending from a terminal 6c provided to the lid 5.

Next, the user inputs, to the allergen inactivation device 100a, the information (that is, the instruction information) regarding instructions on the type of the allergen, the amount of the sample solution 9a, the process completion time, the clock time of the completion, or the degree of inactivation (reduction), for example.

Next, the operation of the allergen inactivation device 100a is described. The controller 30 sets the conditions of applying voltage to the electrodes of the voltage application unit 10a when the instruction information is input by the user (step S101). When setting the conditions, the controller 30 derives the voltage application conditions from the input instruction information and outputs to the power supply 20 a control signal that controls the voltage application by the power supply 20 under the derived voltage application conditions. Note that, in step S101, the controller 30 may acquire a program number and set the voltage application conditions when the program number associated with the voltage application conditions is selected by the user.

Next, the power supply 20 that has acquired the control signal output from the controller 30 starts applying voltage to the electrodes pursuant to the control signal (step S102). For example, the power supply 20 applies voltage between the working electrode 1a and the counter electrode 3 of the voltage application unit 10a, and controls the potential difference between the working electrode 1a and the reference electrode 2 to a particular value (for example, a value greater than or equal to -1.0 V and less than or equal to 0 V). In other words, the power supply 20 applies the voltage such that the voltage applied to the working electrode 1a with respect to the reference electrode 2 (0 V) is greater than or equal to -1.0 V and less than or equal to 0 V. Here, the reference electrode 2 is, for example, a silver/silver chloride electrode. As a result, electrons are donated from the working electrode 1a to the oxidized fusion protein in the sample solution 9a. As a result, the oxidized fusion protein is reduced into a reduced fusion protein (step S103). Next, the reduced fusion protein reduces the allergen in the sample solution 9a and becomes inactivated (step S104). While the voltage is applied from the power supply 20 to the electrodes, steps S103 and S104 are repeated. Here, in step S103, first, the mediator is reduced from the oxidized form to the reduced form, and by the reducing power of the mediator, the thioredoxin reductase and the thioredoxin of the fusion protein are reduced from the oxidized form to the reduced form sequentially in this order.

Next, the controller 30 determines whether or not the process under the set conditions is completed (step S105). The set conditions are, for example, the duration (time) of the voltage application or the number of times voltage is applied (for example, for the pulsed voltage). Next, when it is determined that the process under the set conditions is not completed (No in step S105), the controller 30 commands the power supply 20 to continue voltage application (step S106). Next, until a next determination (step S105) is made, steps S103 and S104 are repeated. Meanwhile, when it is determined that the process under the set conditions is completed (Yes in step S105), the controller 30 commands the power supply 20 to end voltage application (step S 107). As a result, the inactivation of the allergen in the sample solution 9a ends.

Note that the aforementioned preparation step is described as an example performed by the user; alternatively, the preparation step may be performed by the allergen inactivation device 100a. In such a case, the allergen inactivation device 100a may further include an introduction unit (not illustrated), a recovery unit (not illustrated), an introduction port (not illustrated), and a discharge port (not illustrated). For example, the introduction unit may introduce a sample that can contain an allergen into the cell 4 through the introduction port formed in the cell 4. In addition, for example, the recovery unit may recover the sample solution 9a containing the inactivated allergen to the outside of the cell 4 through the discharge port formed in the cell 4.

### [4. Examples]

In the present examples, a fusion protein containing K199R FTRB-TrxY2 (Example 1), a fusion protein containing K199R/K212R/K215R FTRB-TrxY2 (Example 2), and a fusion protein containing K30R/K32R/K34R/K104R FTRA2 and K199R/K212R/K215R FTRB-TrxY2 (Example 3) were prepared. As Comparative Example, a fusion protein not subjected to amino acid substitution was prepared.

The fusion proteins were prepared as follows. An expression vector of a prepared fusion protein was incorporated into Escherichia coli BL21(DE3) (available from NIPPON GENE CO., LTD.), and the resulting Escherichia coli was precultured overnight in an LB medium at 37°C. The precultured Escherichia coli in an appropriate amount was inoculated into MagicMedia E. coli Expression Medium (available from Invitrogen) and cultured overnight at 16°C to express a fusion protein in the bacteria. Subsequently, the cultured bacteria were harvested by centrifugation. The harvested Escherichia coli was suspended in xTractor Buffer (Clontech) and incubated for 10 minutes at 4°C by using a rotator. Centrifugation was conducted at 12,000 × g and 4°C for 20 minutes to remove cell fragments. The expressed fusion protein was purified from the supernatant after the centrifugation by Ni²⁺ affinity chromatography.

In order to examine the effects brought about by the amino acid substitution, enzyme electrodes in which various purified fusion proteins were immobilized through remaining lysine residues were prepared, and, these enzyme electrodes were brought into contact with a sample solution containing an allergen, followed by voltage application, to examine the degradation rate (digestibility) of the allergen. In the description below, a sample solution prepared by dissolving a disulfide bond-containing allergenic protein serving as an allergen in a phosphate buffered saline (PBS) having a pH of 7.4 was used.

First, a fusion protein solution containing a fusion protein and a mediator was prepared. Next, 25 µL of this fusion protein solution was mixed with 4.1 µL of a 20% (w/v) poly L-lysine solution, 4.4 µL of a 2.5% glutaraldehyde solution, 7.2 µL of a 10 mM Tris-HCl buffer, 1.5 µL of a 50 mg/mL BSA (bovine serum albumin) solution, and 12.8 µL of distilled water to prepare a solution for immobilization. A base electrode substrate was impregnated with the solution for immobilization and then was left standing still at 4°C for at least 8 hours, followed by drying. As a result, an enzyme electrode (corresponding to the working electrode 1a) in which the fusion protein and the mediator were immobilized on the surface of the base electrode substrate was obtained.

By using a three-electrode system voltage application cell (corresponding to the voltage application unit 10a) similar to the one described in the embodiment and a potentiostat (corresponding to the power supply 20) were used to apply a particular voltage to the sample solution for 0 hours, 4 hours, or 8 hours. An enzyme electrode prepared as described above was used as the working electrode 1a and a Ag/AgCl electrode was used as the reference electrode 2 among the three electrodes. The particular voltage applied to the sample solution was controlled by the potentiostat such that the potential of the working electrode 1a with respect to the reference electrode 2 was to be the reduction potential of the mediator.

The sample solution after a particular time of voltage application was recovered, digestive enzymes were added to the recovered sample solution, and incubation was carried out at 37°C for 2 hours. Next, the sample solution after the incubation and the molecular weight marker were subjected to common sodium dodecyl sulfate-polyacrylamide gel electrophoresis (SDS-PAGE). Next, the gel after the electrophoresis was stained, and the band area and density of the allergenic protein were quantified by image analysis. Here, as the indicator of the residual rate of 100%, the same SDS-PAGE and image analysis were conducted on a sample solution (hereinafter may be referred to as a control sample solution) containing an un-treated allergen not subjected to the degradation treatment on the enzyme electrode.

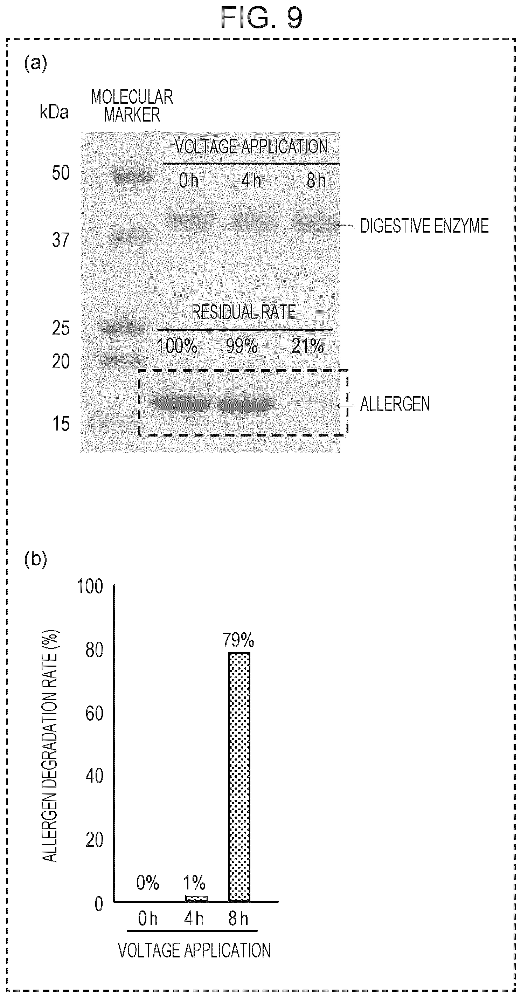

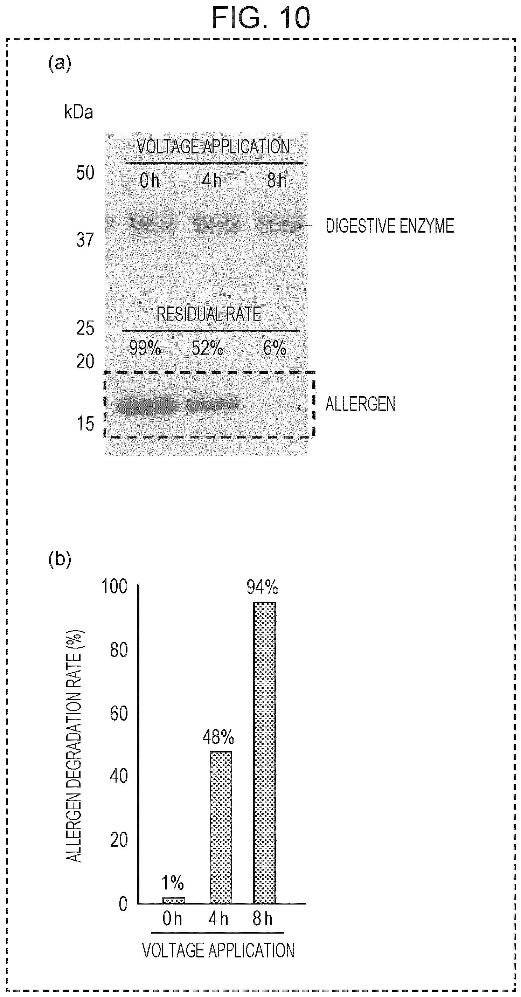

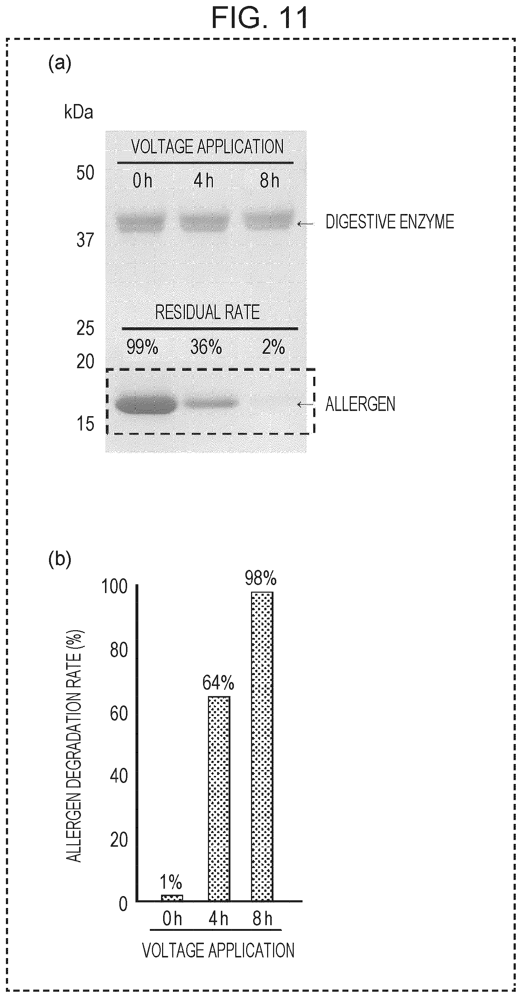

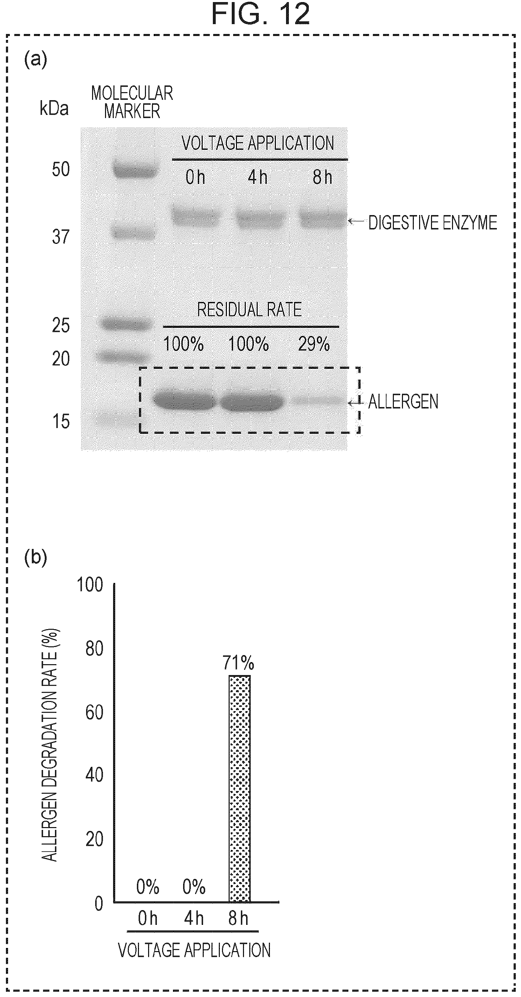

The calculation results of the degradation rates are indicated in Figs. 9 to 12. Fig. 9 is a graph showing the results of allergen degradation in Example 1. Fig. 10 is a graph showing the results of allergen degradation in Example 2. Fig. 11 is a graph showing the results of allergen degradation in Example 3. Fig. 12 is a graph showing the results of allergen degradation in Comparative Example. Note that, in Figs. 9 to 12, the electrophoretic profile after SDS-PAGE is indicated in (a), and the graph of the degradation rate calculation results at the respective time points is indicated in (b).

As indicated in Fig. 9, in Example 1, degradation of the allergen progressed due to the fusion protein containing K199R FTRB-TrxY2, and it was demonstrated that the allergen remained 100% at 0 hour, 99% at 4 hour, and 21% at 8 hour. In terms of the degradation rate, it was demonstrated that the allergen degraded 0% at 0 hour, 1% at 4 hour, and 79% at 8 hour.

As indicated in Fig. 10, in Example 2, degradation of the allergen progressed due to the fusion protein containing K199R/K212R/K215R FTRB-TrxY2, and it was demonstrated that the allergen remained 99% at 0 hour, 52% at 4 hour, and 6% at 8 hour. In terms of the degradation rate, it was demonstrated that the allergen degraded 1% at 0 4 8 hour% at 4 hour, and 94% at 8 hour.

As indicated in Fig. 11, in Example 3, degradation of the allergen progressed due to the fusion protein containing K30R/K32R/K34R/K104R FTRA2 and K199R/K212R/K215R FTRB-TrxY2, and it was demonstrated that the allergen remained 99% at 0 hour, 36% at 4 hour, and 2% at 8 hour. In terms of the degradation rate, it was demonstrated that the allergen degraded 1% at 0 hour, 64% at 4 hour, and 98% at 8 hour.

As indicated in Fig. 12, in Comparative Example, degradation of the allergen progressed due to the fusion protein not subjected to the amino acid substitution, and it was demonstrated that the allergen remained 100% at 0 hour, 100% at 4 hour, and 29% at 8 hour. In terms of the degradation rate, it was demonstrated that the allergen degraded 0% at 0 hour, 0% at 4 hour, and 71% at 8 hour.

Figs. 9 to 12 demonstrated that, compared to Comparative Example, the allergen degradation progressed in a shorter period of time with the enzyme electrodes in which a fusion protein subjected to amino acid substitution was immobilized irrespective of the type of the amino acid substitution. This shows that when a fusion protein having a configuration of amino acids obtained by amino acid substitution involving substituting at least one lysine residue with a non-lysine amino acid is immobilized on an electrode and used as the enzyme electrode, the fusion protein is immobilized on the electrode via more appropriate lysine residues, and thus the present inventors that bonds that are likely to obstruct electron transfer and disulfide bond cleaving actions are less likely to be formed. In other words, it was demonstrated that an enzyme electrode having an enzyme immobilized via more appropriate bonds was realized from the viewpoint of the degradation rate.

In Examples 1 to 3, the descending order for the degradation efficiency was Example 3, Example 2, and Example 1, and it was found that the degradation rate improved with the increase in the number of lysine residues substituted with amino acids. In other words, it was implied that since the effect of individual amino acid substitution was exclusive, the degradation rate improving effect could be increased depending on the combination.

Although the enzyme electrode, the reduction device, the reduction method, the fusion protein (modified protein subjected to amino acid substitution), and the fusion protein production method according to the present disclosure have been described above through the embodiment, the present disclosure is not limited to the embodiment. Various modifications on the embodiment conceived of by a person skilled in the art or any other form configured by combining some constituent elements in the embodiment are also within the scope of the present disclosure without departing from the gist of the present disclosure.

### Industrial Applicability

The present disclosure is applicable to devices that reduce target molecules by using electrodes.

### Reference Signs List

1a working electrode
2 reference electrode
3 counter electrode
4 cell
5 lid
6a, 6b, 6c terminal
7a, 7b, 7c lead
9a sample solution
10a voltage application unit
20 power supply
21, 31 acquisition unit
22 information processing unit
23 voltage control unit
30 controller
32 information processing unit
33 storage unit
34 output unit
100a allergen inactivation device

## Claims

1. An enzyme electrode comprising:
an electrode; and
a modified protein having an amino acid sequence obtained by substituting at least one lysine residue in a fusion protein between a thioredoxin reductase and a thioredoxin with a non-lysine amino acid residue,
wherein
the modified protein is immobilized on a surface of the electrode.

2. The enzyme electrode according to claim 1,wherein
the non-lysine amino acid residue is an arginine residue.

3. The enzyme electrode according to claim 1, wherein
the at least one lysine residue has a side chain oriented outward on a surface of the fusion protein.

4. The enzyme electrode according to claim 1, wherein
the thioredoxin reductase includes a first subunit containing an active center involved in electron transfer and a second subunit different from the first subunit, and
the at least one lysine residue is contained in amino acid residues constituting the second subunit.

5. The enzyme electrode according to any one of claims 1 to 4, wherein
the fusion protein contains a first lysine residue and a second lysine residue, and
the first lysine residue is substituted with the non-lysine amino acid residue, and the modified protein is immobilized on the surface of the electrode via the second lysine residue.

6. The enzyme electrode according to claim 5, wherein
one of the second lysine residue is disposed on a side far from the thioredoxin in the thioredoxin reductase.

7. The enzyme electrode according to claim 5, wherein
one of the second lysine residue is disposed on a side closer to an active center involved in electron transfer in the thioredoxin reductase.

8. A reduction device comprising:
the enzyme electrode according to claim 1; and
a power supply that applies voltage to the electrode,
wherein
a sample containing a target molecule is brought into contact with the enzyme electrode and the target molecule is reduced while applying voltage to the electrode.

9. A reduction method comprising:
bringing a sample that can contain a target molecule into contact with the enzyme electrode according to claim 1; and
reducing the target molecule while applying voltage to the electrode.

10. A modified protein comprising:
an amino acid sequence obtained by substituting at least one lysine residue in a fusion protein between a thioredoxin reductase and a thioredoxin with a non-lysine amino acid residue.

11. A modified protein production method comprising:
preparing a fusion protein between a thioredoxin reductase and a thioredoxin; and
substituting at least one lysine residue of the fusion protein with a non-lysine amino acid residue.
